# EUROPÄISCHE PATENTANMELDUNG

(11) **EP 4 670 765 A1**
(43) Veröffentlichungstag der Anmeldung: **31.12.2025**
(21) Anmeldenummer: 24185139.3
(22) Anmeldetag: 27.06.2024
(51) Int. Cl.: A61M 15/00, B65D 83/20

(54) **AUSTRAGKOPF UND FLÜSSIGKEITSSPENDER FÜR DIE NASALE APPLIKATION VON FLÜSSIGKEIT, VERFAHREN ZUR HERSTELLUNG EINES AUSTRAGKOPFES**

(71) Anmelder: Aptar Radolfzell GmbH, 78315 Radolfzell (DE)
(72) Erfinder: GREINER-PERTH, Jürgen, 78244 Gottmadingen (DE)
(74) Vertreter: Witte, Weller und Partner Patentanwälte mbB Stuttgart

(57) **Zusammenfassung**

Es werden Maßnahmen vorgeschlagen, um solche Austragköpfe preisgünstig herstellen zu können. Insbesondere wird hierzu ein Austragkopf (10) vorgeschlagen, der über eine Basiseinheit (20) mit einer Kopplungseinrichtung (24) verfügt, mittels derer der Austragkopf (10) am Druckspeicher (110) befestigbar ist. Dieser Austragkopf (10) verfügt über eine Betätigungseinheit (50), die einen in einer Applikatorachse (4) erstreckten länglichen Nasalapplikator (60) und eine Betätigungsfläche (70) aufweist und die mittels einer Scharniereinrichtung (30, 90) um eine Schwenkachse (6) schwenkbar an der Basiseinheit (20) angebracht ist, so dass die Betätigungseinheit (50) zum Zwecke des Öffnens eines Auslassventils (112) des Druckspeichers (110) in einer schwenkenden Betätigungsrichtung niederdrückbar ist. Es wird insbesondere vorgeschlagen, Bauteile (22, 52) dieses Austragkopfes mittels einachsig trennbarer Spritzgussformen herzustellen.

## Beschreibung

### ANWENDUNGSGEBIET UND STAND DER TECHNIK

Die Erfindung betrifft einen Austragkopf für die nasale Applikation von Flüssigkeiten aus einem Druckspeicher. Die Erfindung betrifft daneben auch einen Flüssigkeitsspender mit einem solchen Austragkopf sowie Verfahren zur Herstellung eines solchen Austragkopfes.

Gattungsgemäße Austragköpfe sind zum Aufsetzen auf einen Druckspeicher vorgesehen. Sie verfügen über eine Basiseinheit zur zumeist rastenden Verbindung mit dem Druckspeicher. Der Druckspeicher ist üblicherweise als Kunststoff- oder Metallbehälter mit einem innenvolumen von 500 ml oder weniger ausgebildet und weist ein integriertes Auslassventil auf, welches von außen mittels des angebrachten Austragkopfes kraftbeaufschlagt und hierdurch geöffnet werden kann. Im Druckspeicher wird die auszutragende Flüssigkeit unter Druck vorgehalten, üblicherweise unter einem Überdruck von mindestens 1 bar.

Zum Zweck der Ventilöffnung weisen gattungsgemäße Austragköpfe eine Betätigungseinheit auf, die über einen Nasalapplikator und eine Betätigungsfläche verfügt. Wird die Betätigungseinheit niedergedrückt, so wird die Betätigungskraft auf das Auslassventil übertragen und öffnet dieses. Solange die Betätigungseinheit niedergedrückt wird, findet ein Austrag aus einer Austragöffnung am Nasalapplikator statt.

Ein erfindungsgemäßer Austragkopf findet zur Applikation von Flüssigkeit in die Nase des Nutzers Verwendung, insbesondere in Form eines Sprühnebels aus feinen Tröpfchen. Insbesondere dienen gattungsgemäße Austragköpfe dem Austrag von Salzlösung in die Nasenlöcher, um Atembeschwerden zu lindern.

Im Stand der Technik sind beispielsweise aus dem Dokument EP 3412325 A1 gattungsgemäße Austragköpfe bekannt geworden. Der Nachteil bekannter Gestaltungen wie der genannten liegt nicht in der Funktionsweise, sondern primär in der recht aufwändigen Herstellung. Dadurch dass der Nasalapplikator üblicherweise aus Komfortgründen gegenüber einer Mittelachse der Basiseinheit schräggestellt ist, wird der Spritzgussvorgang erschwert. Es finden bislang vergleichsweise aufwändige Werkzeuge Verwendung, die aufgrund von Hinterschneidungen an den Einzelbauteilen des Austragkopfes mit Kernen oder Schiebern versehen werden müssen.

### AUFGABE UND LÖSUNG

Aufgabe der Erfindung ist es, einen Austragkopf der gattungsgemäßen Art bereitzustellen, der in Hinblick auf seine Herstellbarkeit gegenüber bekannten Gestaltungen von Vorteil ist.

Zur Lösung dieser Aufgabe wird ein Austragkopf für die nasale Applikation von Flüssigkeit aus einem Druckspeicher vorgeschlagen, der über eine Basiseinheit und eine Betätigungseinheit verfügt. Diese beiden Teileinheiten sind als getrennte Teileinheiten ausgebildet, die in nachfolgend beschriebener Weise miteinander verbunden sind.

Die Basiseinheit weist eine Kopplungseinrichtung zur Ankopplung an den Druckspeicher auf. Insbesondere kann es sich dabei um eine Kopplungseinrichtung zur Verschnappung mit dem Druckspeicher handeln. Die Kopplungseinrichtung kann durch einen umlaufenden Schnappkragen oder auch durch eine Mehrzahl von Schnapphaken an der Basiseinheit gebildet sein. Auch grundsätzlich andere Konzepte der Kopplungseinrichtungen sind denkbar wie insbesondere die Verwendung einer Gewindeverbindung. Die Basiseinheit ist vorzugsweise durch ein einziges Bauteil gebildet, welches mittels Kunststoff-Spritzguss hergestellt ist.

Die Betätigungseinheit weist einen Nasalapplikator und eine Betätigungsfläche auf. Sie wird durch mindestens ein Bauteil gebildet, insbesondere vorzugsweise durch zwei Bauteile, nämlich ein Außenbauteil und ein Innenbauteil. Der Nasalapplikator ist dafür vorgesehen, in die Nasenlöcher des Benutzers eingeschoben zu werden. Er weist hierfür vorzugsweise eine insgesamt längliche Form auf, die ausgehend von der Betätigungsfläche bis zu einer Austragöffnung am distalen Ende des Applikators länger ist als der mittlere Durchmesser des Nasalapplikators. Der Nasalapplikator definiert eine Applikatorachse entlang seiner Mittelachse. Ist aufgrund der Formgebung des Nasalapplikators keine eindeutige Mittelachse festzulegen, so definiert die Entformungsrichtung bei der Spritzgussherstellung die Applikatorachse im Sinne dieser Erfindung.

Die Applikatorachse ist vorzugsweise gegenüber einer Mittelachse der Basiseinheit und damit einer Mittelachse des Druckspeichers schräggestellt, wobei bezogen auf einen unbetätigten Zustand der Winkel zwischen der Mittelachse und der Applikatorachse vorzugsweise mindestens 10° beträgt, insbesondere vorzugsweise mindestens 15°. Der Winkel beträgt jedoch vorzugsweise nicht mehr als 35°. Diese schräge Ausrichtung des Nasalapplikators ermöglicht eine angenehme Handhabung bei der Nutzung des Flüssigkeitsspenders.

Die Betätigungsfläche ist ortsfest zum Nasalapplikatorvorgesehen und insbesondere versetzt hierzu neben dem proximalen Ende des Nasalapplikators angeordnet. Die Betätigungsfläche wird im Zuge der Betätigung gegenüber der Basiseinheit verlagert, wobei der Nasalapplikator hiermit gemeinsam bewegt wird. Die Betätigungsfläche ist vorzugsweise mit Erhebungen versehen, um einen sicheren Griff und eine sichere Handhabung zu ermöglichen.

Im Falle einer mehrteiligen Betätigungseinheit sind der Nasalapplikator und die Betätigungsfläche vorzugsweise an einem gemeinsamen einstückigen Bauteil vorgesehen.

Erfindungsgemäß ist vorgesehen, dass die Betätigungseinheit mittels einer Scharniereinrichtung um eine Schwenkachse schwenkbar an der Basiseinheit angebracht ist, so dass die Betätigungseinheit zum Zwecke des Öffnens eines Ventils des Druckspeichers in einer schwenkenden Betätigungsrichtung gegenüber der Basiseinheit niederdrückbar ist.

Es besteht somit bei einemerfindungsgemäßen Austragkopf keine einstückige Verbindung zwischen der Basiseinheit und der Betätigungseinheit. Stattdessen wird die genannte Scharniereinrichtung durch ein Bauteil der Basiseinheit und ein Bauteil der Betätigungseinheit gebildet.

Diese Bauweise geht damit einher, dass der Austragkopf aus mindestens zwei Teilen gebildet ist, die separat hergestellt worden sind, insbesondere jeweils mittels eines Kunststoffspritzguss-Verfahrens. Dies ermöglicht es, diese beiden Bauteil jeweils getrennt in Hinblick auf den Spritzgussvorgang zu optimieren, wie im Weiteren noch erläutert wird. Darüber hinaus kann die Scharniereinrichtung auch eine einfache Form der Montage ermöglichen. So kann im Zuge der Montage zunächst die Scharniereinrichtung gefügt werden und dann eine hierdurch geführte Relativbewegung der beiden damit gefügten Bauteile relativ zueinander in ihre Sollstellung erfolgen.

Vorzugsweise ist vorgesehen, dass die Schwenkachse und die Betätigungsfläche gegenüber dem Nasalapplikator an entgegengesetzten Seiten der Betätigungseinheit angeordnet sind. Die Schwenkachse ist hierbei insbesondere vorzugsweise nahe dem Nasalapplikator auf der der Betätigungsfläche abgewandten Seite der Betätigungseinheit vorgesehen. Hierdurch wird aufgrund der Schwenkbeweglichkeit unter anderem erreicht, dass ein vergleichsweise großer Betätigungsweg an der Betätigungsfläche zu einer demgegenüber verringerten Bewegung des Nasalapplikators führt. Dies ist von Vorteil, damit der Nutzer nicht durch die Betätigungsbewegung den Nasalapplikator wieder aus dem Nasenloch herauszieht.

Vorzugsweise weist die Basiseinheit ein Basisbauteil auf, welches mindestens die Kopplungseinrichtung sowie eine basiseinheitsseitige Scharnierteileinrichtung umfasst. Insbesondere vorzugsweise kann dieses Basisbauteil das einzige Bauteil der Basiseinheit sein und diese somit in Gesamtheit bilden.

Das Basisbauteil weist vorzugsweise eine Bauteilgeometrie auf, die bezogen auf einer Trennrichtung, die der Mittelachse der Basiseinheit entspricht, frei ist von nicht zwangsentformbaren Hinterschneidungen. Dies bedeutet, dass das Basisbauteil mittels eines Werkzeugs herstellbar ist, welches ausschließlich Spritzgussformsegmente umfasst, die entlang einer einheitlichen Schließ- und Trennrichtung gegeneinander beweglich sind. Derartige Werkzeuge sind erheblich einfacher herzustellen als Werkzeuge, bei denen Schieber zur Herstellung von Hinterschneidungen vorgesehen sind, die entlang einer von einer Hauptschließrichtung/Haupttrennrichtung abweichende Bewegungsrichtung beim Entformen zu bewegen sind. Entsprechende Werkzeuge mit einheitlicher Schließ- und Trennrichtung sind nicht nur preisgünstiger, sondern im Betrieb auch wartungsärmer. Sie erlauben zusätzlich noch kürzere Taktzeiten.

Das Basisbauteil muss zum Zwecke der einfachen Entformbarkeit nicht vollständig hinterschneidungsfrei sein. Im Gegenteil ist es aufgrund der Kopplungseinrichtung auf Seiten der Basiseinheit schwierig, Hinterschneidungen vollständig zu vermeiden. Allerdings ist gemäß dieser Weiterbildung vorgesehen, dass nur solche Hinterschneidungen am Basisbauteil vorhanden sind, die zwangsentformbare Hinterschneidungen darstellen. Zwangsentformbare Hinterschneidungen bezeichnen dabei Bereiche des Bauteils, die einer Entformung zwar im Wege sind, die aber durch zerstörungsfreie Verformung des Bauteils dennoch die einachsige Entformung ermöglichen. Die Entformung erfolgt also bei gleichzeitigem elastischem Nachgeben des Materials des Basisbauteils im Bereich der zwangsentformbaren Hinterschneidungen.

Ebenso wie das Basisbauteil ist es auch im Falle der Betätigungseinheit und insbesondere im Falle eines Außenbauteils der Betätigungseinheit von besonderem Vorteil, wenn dieses Bauteil eine Bauteilgeometrie aufweist, die bezogen auf eine Trennrichtung, die der der Applikatorachse entspricht, frei von nicht zwangsentformbaren Hinterschneidungen ist.

Wenn beide Bauteile, also das Basisbauteil der Basiseinheit und das Außenbauteil der Betätigungseinheit, derart ausgebildet sind, so lassen sich diese beiden Hauptbauteile sehr preisgünstig und bei kurzen Taktzeiten herstellen.

Die Basiseinheit, insbesondere das genannte zwangsentformbare Basisbauteil, weist die basiseinheitsseitige Scharnierteileinrichtung auf. Diese kann vorzugweise derart ausgebildet sein, dass sie zwei äußere Lagerschalenabschnitte und unterhalb dieser äußeren Lagerschalenabschnitten und bezogen auf eine Querrichtung zur Applikatorachse zwischen den äußeren Lagerschalenabschnitten einen gegenüberliegenden inneren Lagerschalenabschnitt aufweist. Es wird also vorgeschlagen, dass mindestens drei Lagerschalenabschnitte vorgesehen sind, die versetzt im Wechsel einen Bereich für einen Achsabschnitt von oben und unten umfassen. Durch ihre versetzte Anordnung quer zur Mittelachse und somit quer zur bevorzugten Trennrichtung des Spritzgusswerkzeugs können diese Lagerschalenabschnitte hinterschneidungsfrei hergestellt werden, indem zwei Spritzgussformsegmente jeweils jeden der Lagerabschnitte von oben und unten bezüglich der jeweiligen Form definieren.

Um diese vorteilhafte Gestaltung zu erzielen, ist vorzugsweise vorgesehen, dass zum Zwecke der Entformbarkeit des Basisbauteils bei der Spritzgussherstellung bezogen auf die Mittelachse oberhalb des inneren Lagerschalenabschnitts und unterhalb der äußeren Lagerschalenabschnitte keine Abschnitte des Basisbauteils vorhanden sind. Die Lagerschalenabschnitte bilden also bezogen auf die Trennrichtung jeweils obere und untere Abschlussflächen des Basisbauteils.

Betreffend die betätigungseinheitsseitige Scharnierteileinrichtung ist zum Zwecke der einfachen Herstellbarkeit vorzugsweise vorgesehen, dass die Scharnierteileinrichtung einen zentralen Achsträger aufweist, der an seiner Unterseite über eine Lagerkontaktfläche verfügt, und beidseitig an diesem Achsträger einander gegenüberliegend zwei Achsabschnitte aufweist, die an ihrer Oberseite über eine Lagerkontaktfläche verfügen.

Die betätigungseinheitsseitige Scharnierteileinrichtung ist hierdurch passend zur genannten Bauweise der basisseitigen Scharnierteileinrichtung ausgebildet. Die Unterseite des Achsträgers bildet den Achsbereich, der in Kontakt mit dem mittleren Lagerschalenabschnitt des Basisteils steht. Die beiden Achsabschnitte, die beidseitig am Achsträger vorgesehen sind und frei hiervon hervorragen, bilden mit ihren Oberseiten die Lagerkontaktflächen, die zumindest abschnittsweise an den beiden äußeren Lagerschalenabschnitten anliegen.

Vorzugsweise ist vorgesehen, dass zum Zwecke der einfachen Entformbarkeit des Außenbauteils, an dem die betätigungseinheitsseitige Scharnierteileinrichtung vorgesehen ist, bezogen auf die Applikatorachse oberhalb der Oberseite der Achsabschnitte unterhalb der Unterseite des Achsträgers keine Abschnitte des Außenbauteils vorhanden sind. Die Ober- und Unterseiten des Achsträgers und der Achsabschnitte bilden also bezogen auf die Trennrichtung jeweils obere und untere Abschlussflächen des Außenbauteils.

Die Lagerschalenabschnitte eines erfindungsgemäßen Austragkopfes weisen auf ihrer zur Achse hinweisenden Seite vorzugsweise zumindest abschnittsweise eine kreiszylinderabschnittsförmige oder abschnittsweise rotationssymmetrische Fläche auf, die gemeinsam mit abschnittsweise kreiszylindrischer Formgebung der Achsabschnitte und der Unterseite des Achsträgers ein reibungs- und ruckelarmes Schwenken ermöglichen.

Die beschriebene Gestaltung der Lagerschalenabschnitte, des Achsträgers und der Achsabschnitte stellt demnach eine Möglichkeit dar, um das Außenbauteil der Betätigungseinheit und/oder das Basisbauteil im Spritzgussverfahren ohne Schieber herstellen zu können.

Auch im Übrigen sind die genannten beiden genannten Bauteile vorzugsweise dahingehend gestaltet, dass eine Herstellung mit einem einachsigen Spritzgusswerkzeug erfolgen kann, also mit einer aus mindestens zwei Spritzgussformsegmenten zusammengesetzten kavitätsbildenden Form, deren Spritzgussformsegmente, die die Kavität begrenzen, zur Entformung nur in einer Trennrichtung voneinander beabstandet werden.

Insbesondere kann die Betätigungseinheit, insbesondere das Außenbauteil der Betätigungseinheit, an einer der Scharniereinrichtung abgewandten Seite eine Schürze aufweisen, die sich in Richtung der Applikatorachse erstreckt. Durch die Erstreckung in Richtung der Applikatorachse stellt die Entformung dieser Schürze kein Problem dar.

Weiterhin vorzugsweise weist die Betätigungseinheit mindestens einen nach außen ragenden Anschlagsflügel auf, der die Beweglichkeit der Betätigungseinheit entgegen der Betätigungsrichtung durch Anschlagen an einer korrespondierenden Haltefläche der Basiseinheit limitiert. Vorzugsweise sind mindestens zwei Anschlagsflügel vorgesehen, die einander gegenüberliegend an beiden Seiten der Betätigungseinheit vorgesehen sind. Der oder die Anschlagsflügel weisen vorzugsweise eine Anschlagsfläche auf, deren Normalenvektor nicht mit der Applikatorachse übereinstimmt. Dennoch stehen die Anschlagsflügel einer Verwendung eines einachsigen Spritzgusswerkzeugs nicht im Wege, wenn zum Zwecke der Gewährleistung der einachsigen EntformbarkeitdesAußenbauteils bezogen auf die Applikatorachse oberhalb und unterhalb der des Anschlagsflügels keine Abschnitte des Außenbauteils vorhanden sind.

Der mindestens eine Anschlagsflügel dient während der Benutzung des Flüssigkeitsspenders dem Zweck, eine Endstellung zu definieren, bis in die die Betätigungseinheit nach erfolgtem Austrag zurückschwenken kann. Zusätzlich können die Anschlagsflügel dem Zweck dienen, Montagekräfte während der Montage aufzunehmen, wie im Weiteren noch erläutert wird.

Wie oben bereits beschrieben, weist ein erfindungsgemäßer Austragkopf eine Scharniereinrichtung auf, die die Schwenkbeweglichkeit von Basiseinheit und Betätigungseinheit ermöglicht. Grundsätzlich ist die eingangs beschriebene Zuordnung der beiden Scharnierteileinrichtungen auf die Basiseinheit und die Betätigungseinheit nicht zwingend.

Verallgemeinert wird die folgende Bauweise vorgeschlagen, bei der nicht festgelegt ist, welche der Scharnierteileinrichtungen an welcher Einheit vorgesehen ist. Gemäß dieser Bauweise weist die Scharniereinrichtung eine erste Scharnierteileinrichtung und eine zweite Scharnierteileinrichtung auf.

Die erste Scharnierteileinrichtung bildet ein Scharnierlager und weist mindestens zwei äußere Lagerschalenabschnitte und einen hierzu in Richtungder Applikatorachse gegenüberliegend angeordneten inneren Lagerschalenabschnitt auf. Die zweite Scharnierteileinrichtung bildet eine Scharnierachse und weist einen zentrischen Achsträger auf, der am inneren Lagerschalenabschnitt gelagert ist sowie zwei hiermit fluchtende frei hervorstehende Achsabschnitte, die an den äußeren Lagerschalenabschnitten gelagert sind.

Unabhängig davon, ob diese oder eine andere Bauweise der Scharniereinrichtung genutzt wird, ist es bevorzugt, dass eine Fügerichtung der Scharniereinrichtung, in Richtung derer die Scharnierteileinrichtungen der Betätigungseinheit und der Basiseinheit koppelbar sind, gegenüber einer Mittelachse der Basiseinheit angewinkelt ist, vorzugsweise um einen Winkel von mindestens 60°, insbesondere vorzugsweise um einen Winkel von mindestens 80°, insbesondere um in etwa 90°.

Die Mittelachse der Basiseinheit definiert eine Richtung, die mit der Montagerichtung des Austragkopfes auf dem Druckspeicher übereinstimmt. Wenn die Scharniereinrichtung, wie oben vorgeschlagen, eine Fügerichtung aufweist, die von der Montagerichtung abweicht, so ist dies der Montage dienlich, da die Gefahr verringert wird, dass beim Aufdrücken des Austragkopfes auf den Druckspeicher die Scharnierteileinrichtungen sich voneinander trennen. Diese bevorzugte Fügerichtung der Scharnierteileinrichtung und der oben genannte Anschlagsflügel gewährleisten gemeinsam, dass bei der Montage des Austragkopfes auf einem Druckspeicher die Betätigungseinheit gegenüber der Basiseinheit eine definierte Position einnimmt und dabei auch Kräfte in Fügerichtung aufnehmen kann.

Insbesondere vorzugsweise ist in Hinblick auf die Scharniereinrichtung vorgesehen, dass die Lagerschalenabschnitte sich von einer Innenseite des Basisbauteils im Wesentlichen orthogonal zur Mittelachse des Basisbauteils nach innen erstrecken.

Die Scharniereinrichtung ist vorzugsweise mit etwas Spiel ausgelegt. Dies dient nicht nur der leichtgängigen Beweglichkeit der Betätigungseinheit gegenüber der Basiseinheit, sondern kann auch eine gewünschte translative Relativbeweglichkeit ermöglichen. Vorzugsweise sind zu diesem Zweck die Scharnierteileinrichtungen derart geformt, dass sie in einem gekoppelten Zustand verformungsfrei gegeneinander in Fügerichtung begrenzt beweglich sind, vorzugsweise um eine Verlagerungsstrecke von mindestens 0,2 mm, insbesondere um mindestens 0,4 mm.

Diese Relativbeweglichkeit führt dazu, dass das Zusammenwirken der Betätigungseinheit mit dem druckspeicherseitigen Ventil verbessert wird. Die Schwenkbeweglichkeit der Betätigungseinheit, die vorliegend vorgeschlagen wird, führt dazu, dass der Abstand zwischen dem Ventil und einem hierauf wirkenden Teilabschnitt der Betätigungseinheit, insbesondere einem Anschlussstutzen an der Betätigungseinheit, der an einen Ventilkörper des Auslassventils ankoppelt, quer zur Mittelachse der Basiseinheit veränderlich ist, was grundsätzlich die Gefahr eines Verkantens mit sich bringt. Die Verlagerbarkeit, die die Scharniereinrichtung gemäß dieser Weiterbildung ermöglicht, kann dies kompensieren und dazu führen, dass der Abstand zwischen dem Ventil und dem hierauf wirkenden Teilabschnitt der Betätigungseinheit in Querrichtung in geringerem Maße oder gar nicht veränderlich ist. Ein störungsfreierer Betrieb wird hierdurch ermöglicht.

Vorzugweise weist ein erfindungsgemäßer Austragkopf einen Betätigungsschutz auf, der einen an der Betätigungseinheit oder der Basiseinheit einstückig angeformten Schutzabschnitt umfasst. Der Schutzabschnitt weist mindestens einen zerstörbaren Teilabschnitt auf, der durch eine Betätigungsverlagerung der Betätigungseinheit gegenüber der Basiseinheit zerstörbar ist.

Ein solcher Schutzabschnitt kann mehreren Zwecken dienen. Er kann eine ungewünschte Betätigung des Flüssigkeitsspenders, beispielsweise im Reisegepäck, verhindern und er kann anzeigen, dass eine Erstbenutzung noch nicht stattgefunden hat. Im vorliegenden Falle ist der Schutzabschnitt vorzugsweise einstückiger Teil des Außenbauteils oder des Basisbauteils. Er kann einen oder mehrere zerstörbare Teilabschnitte aufweisen, die bei Inbetriebnahme des Flüssigkeitsspenders zerstört werden, beispielsweise in Form von Kunststoffbrücken, die bei Erstbetätigung durchtrennt werden.

Vorzugsweise ist der Schutzabschnitt derart ausgebildet, dass nach Zerstörung des mindestens einen zerstörbaren Teilabschnitts keine vollständige Trennung eines Teiles des Schutzabschnitts vom Basisbauteil bzw. einem der Bauteile der Betätigungseinheit eintritt. Insbesondere vorzugweise bildet ein stabiler Fortsatz an der Innenseite eines der Bauteile einen nach innen ragenden Träger aus, zwischen dem und der Innenwandung des Basisbauteils zerstörbare Kunststoffbrücken vorgesehen sind. Bei Erstbetätigung werden die Brücke oder die Brücken zerstört, der Träger mit den getrennten Brücken verbleibt jedoch am Basisbauteil.

Ein Austragkopf erfindungsgemäßer Art hat ohne Berücksichtigung der auf die Basiseinheit aufsetzbaren Schutzkappe mindestens zwei Bauteile. Vorzugsweise jedoch ist die Betätigungseinheit mindestens zweigeteilt, so dass neben einem Außenbauteil auch ein Innenbauteil vorgesehen ist.

Das Außenbauteil umfasst mindestens die Betätigungsfläche, die die betätigungseinheitsseitige Scharnierteileinrichtung sowie einen Hüllenabschnitt des Nasalapplikators einschließlich einer Austragöffnung bildet. Das Innenbauteil ist mit dem Außenteil dauerhaft verbunden, beispielsweise durch eine Klemmverbindung. Es bildet einen Leitungsabschnitt, mittels dessen Flüssigkeit vom Druckspeicherzur Austragöffnung geleitet werden kann. Insbesondere kann das Innenbauteileinen Anschlussstutzen zur Kraftübertragung auf das Auslassventil des Druckspeichers und/oder einen Einsatz, der sich in den Nasalapplikator hinein erstreckt, bilden.

Die Verwendung eines vom Außenbauteil getrennten Innenbauteils vereinfacht die Herstellung, da das Außenbauteil insbesondere durch diese Maßnahme in einer einachsig trennbaren Spritzgussform hergestellt werden kann.

Wie eingangs erläutert, findet der beschriebene Austragkopf als Teil eines Flüssigkeitsspenders Verwendung, der einen Druckspeicher mit integriertem Auslassventil umfasst. Die Betätigungseinheit drückt auf einen Ventilkörper dieses Auslassventils, wenn eine manuelle Betätigung stattfindet. Das Auslassventil öffnet und die Flüssigkeit kann aus dem Druckspeicher in den Austragkopf bis zur Austragöffnung strömen. Der Flüssigkeitsdruck und/oder hierfür angepasste Geometrien vor der Austragöffnung können die Erzeugung eines Sprühstrahls ermöglichen.

Der Flüssigkeitsspender ist vorzugsweise mit einer pharmazeutischen Flüssigkeit befüllt. Hierzu zählen im Sinne der Erfindung auch Salzlösungen, die zur Linderung von Atembeschwerden abgegeben werden können.

Wie bereits im Kontext der Beschreibung des Austragkopfes erläutert, liegen Vorteile der beschriebenen Gestaltung in der Einfachheit bei der Herstellung der Einzelteile und der Handhabung bei der Montage.

Die Erfindung umfasst auch neue Verfahren zur Herstellung der Einzelteile und zur Montage.

In Hinblick auf die Herstellung eines Basisbauteils, welches eine basiseinheitsseitige Scharnierteileinrichtung mit mindestens einem unteren Lagerschalenabschnitt und mindestens einem oberen Lagerschalenabschnitt umfasst, wird vorgeschlagen, dass die Herstellung mittels eines Spritzgussverfahrens erfolgt, bei dem eine Mehrzahl von Spritzgussformsegmenten Verwendung findet, die entlang einer gemeinsamen Schließ- und Trennrichtung verlagerbar sind. Im Zuge der Herstellung gemäß diesem Verfahren werden die Spritzgussformsegmente zur Bildung einer dem Basisbauteil entsprechenden Kavitätzusammengefahren, Kunststoffmaterial wird in die Kavität eingespritzt und nach dem Aushärten werden die Spritzgussformsegmente zum Zwecke der Entformung in der gemeinsamen Öffnungs- und Trennrichtung gegeneinander verlagert (einachsige Spritzgusswerkzeugtrennung).

Die vergleichsweise einfache Form des Basisbauteils und die Gestaltung der Scharnierteileinrichtung gestatten die Verwendung eines solchen einachsigen Spritzgusswerkzeugs.

Vorteilhaft ist es zur Herstellung der Scharnierteileinrichtung, wenn zum Zwecke der Entformung mindestens ein erstes Spritzgussformsegment, welches an eine Oberseite des mindestens einen oberen Lagerschalenabschnitts und an eine obere Innenseite des mindestens einen unteren Lagerschalenabschnitts des Basisbauteils angrenzt, gegenüber mindestens einem zweiten Spritzgussformsegment, welches an eine Unterseite des mindestens einen unteren Lagerschalenabschnitts und eine untere Innenseite des mindestens einen oberen Lagerschalenabschnitts angrenzt, beabstandet wird.

In Hinblick auf die Herstellung eines Außenbauteils, welches eine betätigungseinheitsseitige Scharnierteileinrichtung aufweist, wobei diese Scharnierteileinrichtung über einen zentralen Achsträger verfügt, der an seiner Unterseite über eine Lagerkontaktfläche aufweist und wobei die Scharnierteileinrichtung beidseitig am Achsträger einander gegenüberliegend zwei Achsabschnitte aufweist, die an ihrer Oberseite über eine Lagerkontaktfläche verfügen, wird ebenfalls vorgeschlagen, dass die Herstellung mittels eines Spritzgussverfahrens erfolgt, bei dem eine Mehrzahl von Spritzgussformsegmenten Verwendung findet, die entlang einer gemeinsamen Schließ- und Trennrichtung verlagerbar sind.

Auch hier werden im Zuge der Herstellung die Spritzgussformsegmente zur Bildung einer dem Außenbauteil entsprechenden Kavität zusammengefahren, Kunststoffmaterial wird in die Kavität eingespritzt und nach dem Aushärten werden die Spritzgussformsegmente zum Zwecke der Entformung in der gemeinsamen Schließ- und Trennrichtung gegeneinander verlagert (einachsige Spritzgusswerkzeugtrennung). Zum Zwecke der Entformung wird vorzugsweise mindestens ein erstes Spritzgussformsegment, welches an eine Oberseite der Achsabschnitte angrenzt, gegenüber mindestens einem zweiten Spritzgussformsegment, welches an eine Unterseite des zentralen Achsträgers angrenzt, beabstandet.

Weiterhin wird ein vorteilhaftes Montageverfahren vorgeschlagen. Dieses Montageverfahren dient der Montage eines Austragkopfes mit einer Basiseinheit und einer Betätigungseinheit auf einem Druckspeicher. Dabei weist die Betätigungseinheit mindestens einen nach außen ragenden Anschlagsflügel der beschriebenen Art auf, der die Beweglichkeit der Betätigungseinheit entgegen der Betätigungsrichtung durch Anschlagen an einer korrespondierenden Haltefläche der Basiseinheit limitiert. Weiterhin weist die Betätigungseinheit einen Anschlussstutzen zur Kraftübertragung auf ein Auslassventil des Druckspeichers auf. Dieser Anschlussstutzen ist vorzugsweise vom Auslasskanal durchdrungen.

Während der Montage des Austragkopfes auf den Druckspeicher wird zum einen eine Schnappverbindung zwischen der Kopplungseinrichtung der Basiseinheit mit einer Rastkante des Druckspeichers hergestellt. Zum anderen wird der Anschlussstutzen an das Auslassventil des Druckspeichers angekoppelt, beispielsweise durch Einschieben in einen Ventilkörper des Auslassventils.

Die hierfür erforderliche Montagekraft wird über die Basiseinheit aufgebracht, insbesondere über eine auf der Basiseinheit aufgesetzte Schutzkappe.

Der mindestens eine Anschlagsflügel dient während des Aufdrückens des Austragkopfes auf den Druckspeicher der Kraftübertragung von der Basiseinheit auf die Betätigungseinheit, so dass der Anschlussstutzen der Betätigungseinheit mittels dieser Kraft an das Auslassventil angekoppelt werden kann. Der oder die Anschlagsflügel sind derart ausgelegt, dass die hierbei zu erwartende Kraft ohne Zerstörung des oder der Anschlagsflügel übertragen werden kann.

Insbesondere vorzugsweise ist vorgesehen, dass vor dem Aufschnappen des Austragkopfes auf den Druckspeicher die Scharniereinrichtung gefügt worden ist, wobei vorzugsweise hierdurch die Anschlagsflügel in den Bereich der Halteflächen an der Basiseinheit gelangen. Die Montagekraft kann dann von der Scharniereinrichtung und den Anschlagsflügeln gemeinsam von der Basiseinheit auf die Betätigungseinheit und damit auf den Anschlussstutzen übertragen werden.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen einen erfindungsgemäßen Flüssigkeitsspender in geschnittener und ungeschnitter Darstellung.
Fig. 2 zeigt einen Austragkopf des Flüssigkeitsspenders in separater Darstellung.
Fig. 3 zeigt die Einzelteile des Austragkopfes in einer Explosionsdarstellung.
Fig. 4A bis 4C zeigen Bauteile des Austragkopfes aus weiteren Perspektiven.
Fig. 5 verdeutlicht die Details zur Scharniereinrichtung des Austragkopfes.
Fig. 6A bis 8 verdeutlichen den Spritzgussvorgang für zwei der Bauteile des Austragkopfes sowie den Montagevorgang.

### DETAILLIERTE BESCHREIBUNG DER AUSFÜHRUNGSBEISPIELE

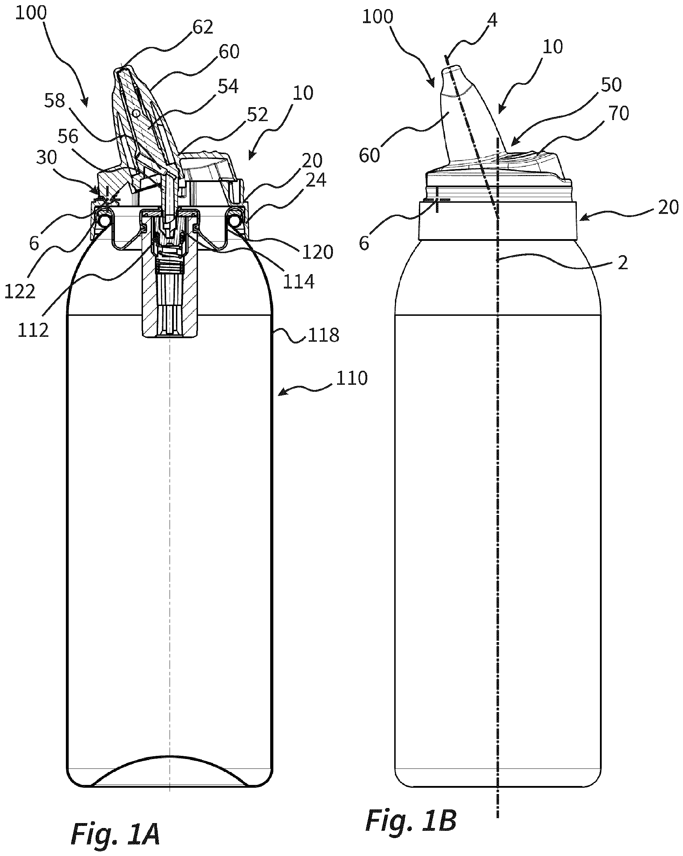

Die Fig. 1A und 1B zeigen eine Gesamtdarstellung eines erfindungsgemäßen Flüssigkeitsspenders 100 in geschnittener und ungeschnittener Darstellung.

Ein erfindungsgemäßer Flüssigkeitsspender 100 gemäß den vorliegenden Ausführungsbeispielen verfügt über einen Druckspeicher 110, dessen Außenflächen durch einen metallischen Speicherkörper 118 und einen teilweise metallischen Deckel 120 gebildet werden. Es könnte sich stattdessen auch um einen Speicherkörper oder einen Deckel aus Kunststoff handeln.

An einer Rastkante 122 des Deckels 120 ist der Austragkopf 10 rastend befestigt. Er verfügt hierfür über eine Basiseinheit 20, die durch ein einstückiges Basisbauteil 22 gebildet ist. Dieses Basisbauteil 22 weist eine ringförmige Außenwandung auf, an deren Innenseite die Kopplungseinrichtung 24 in Form eines umlaufenden oder unterbrochenen Raststeges vorgesehen ist, die mit der Rastkante 122 verschnappt ist.

Am Basisbauteil 22 ist eine Betätigungseinheit50 des Austragkopfes 10 schwenkbar angelenkt. Hierfür sind das Basisbauteil 22 und ein Außenbauteil 52 der Betätigungseinheit 50 mittels einer Scharniereinrichtung aus zwei Scharnierteileinrichtungen 30, 90 miteinander verbunden. Die Scharniereinrichtung definiert die Schwenkachse 6 der Betätigungseinheit 50.

Die Betätigungseinheit 50 bildet mittels ihres Außenbauteils 52 die Außenhülle eines in einer Applikatorachse 4 erstreckten Nasalapplikators 60, an dessen distalem Ende eine Austragöffnung 62 vorgesehen ist. Die Betätigungseinheit 50 bildet weiterhin mittels eines Innenbauteils 54, welches an einer Innenkontur des Nasalapplikators 60 verrastet ist, einen Fluidkanal 58, dersich von einem Anschlussstutzen 56 bis in den Nasalapplikator 60 erstreckt.

Der Anschlussstutzen 56 ragt durch eine zentrische Öffnung des Deckels 120 in einen Ventilkörper 114 des Auslassventils 112 hinein.

Die Betätigung des Flüssigkeitsspenders 100 erfolgt, indem der Benutzer zunächst den Nasalapplikator 60 in ein Nasenloch einführt und dann die Betätigungsfläche 70 manuell niederdrückt. Hierbei verschwenkt die gesamte Betätigungseinheit 50 bezogen auf Fig 1A und 1B im Uhrzeigersinn um etwa 5°. Der Anschlussstutzen 56 drückt auf den Ventilkörper 114 und beabstandet diesen von einer Dichtfläche des Auslassventils, so dass die unter Druck stehende Flüssigkeit aus dem Druckspeicher 110 in den Austragkopf und bis zur Austragöffnung 62 strömt und dort in Form eines Sprühstrahls ausgegeben wird.

Die Bauweise des Austragkopfes 10 ist insbesondere in Hinblick auf einen einfachen und kostengünstigen Herstellungsvorgang optimiert. Der Austragkopf 10 besteht aus den genannten drei Bauteilen, also dem Basisbauteil 22 der Basiseinheit 20 sowie dem Außenbauteil52 und dem Innenbauteil 54 der Betätigungseinheit 50. Zusätzlich kann der Austragkopf über eine Schutzkappe als viertem Bauteil des Austragkopfes 10 verfügen.

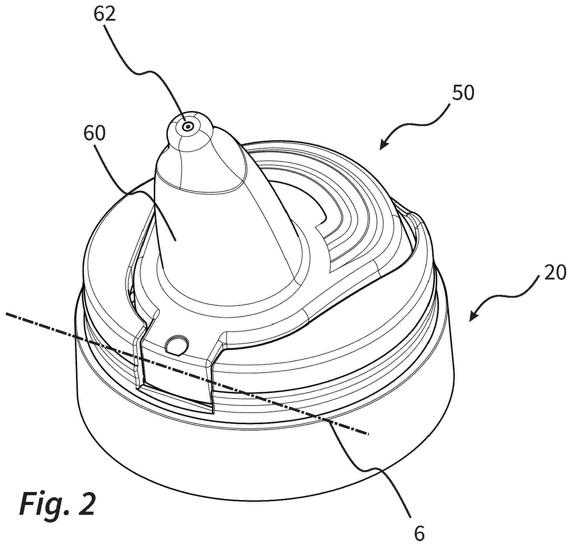

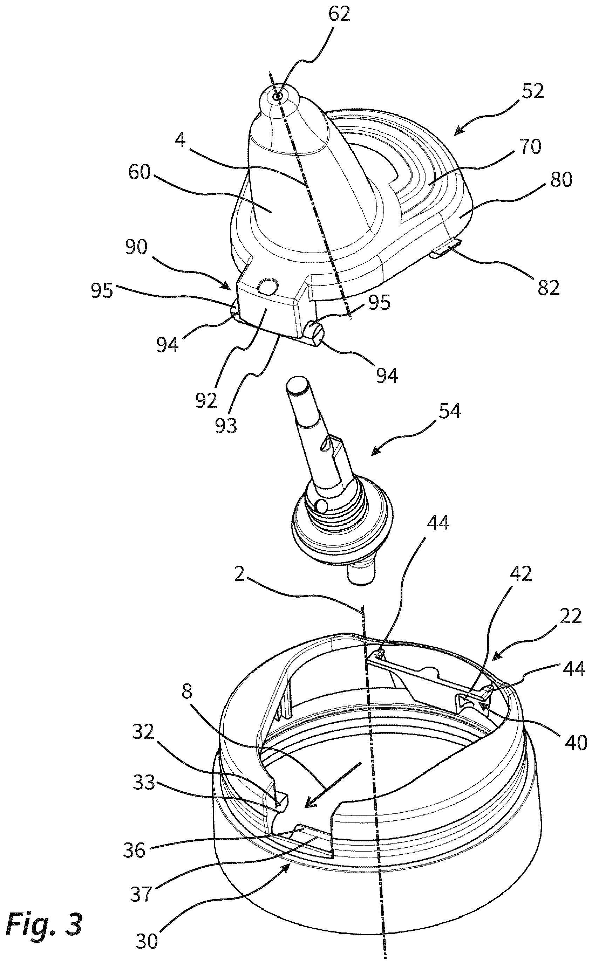

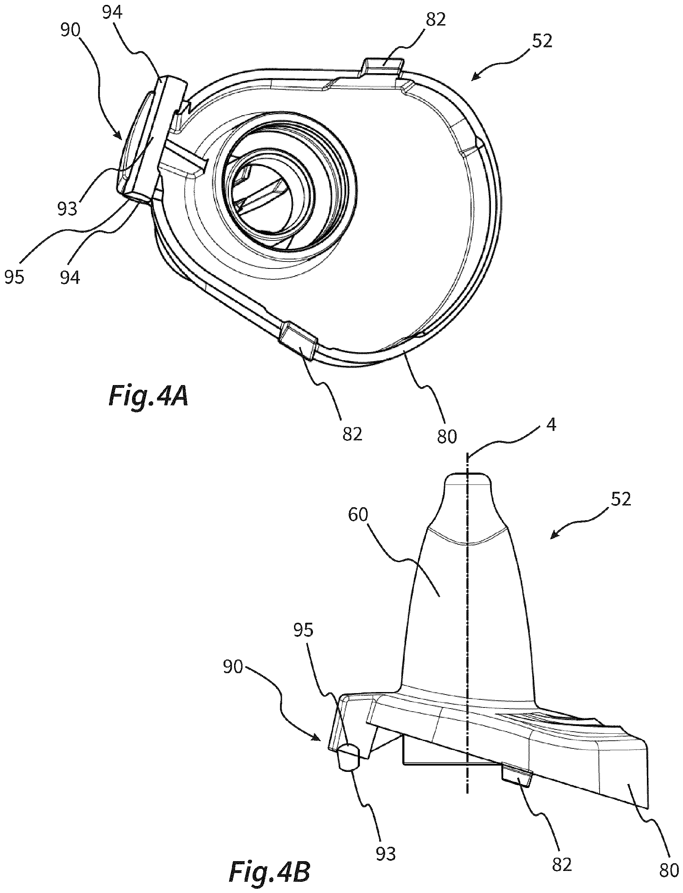

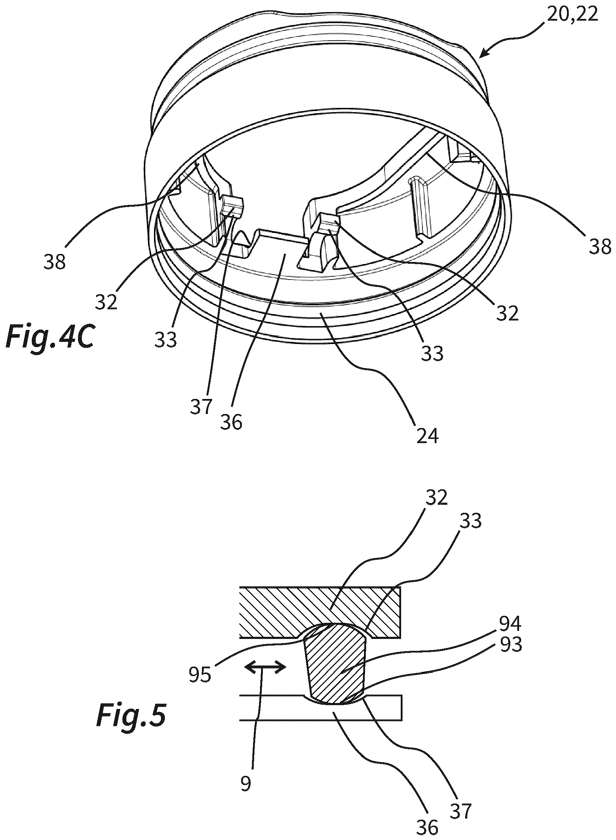

Fig. 2 zeigt nochmals den Austragkopf 10 in separater Darstellung. Fig. 3 zeigt die drei Bauteile 22, 52, 54 des Austragkopfes 10 in einer Explosionsdarstellungen. Die Fig. 4A und 4B zeigen das Außenbauteil 52 in weiteren Perspektiven. Fig. 4C zeigt das Basisbauteil in einer separaten Darstellung. Anhand dieser Figuren werden nachfolgend Details des Austragkopfes 10 erläutert.

Die aus den beiden Scharnierteileinrichtungen 30, 90 am Außenbauteil 52 und dem Basisbauteil 22 gebildete Scharniereinrichtung ist derart gestaltet, dass sie eine besonders einfache Herstellbarkeit der Bauteile als Spritzgussbauteile ermöglicht. Die am Basisbauteil 22 vorgesehene Scharnierteileinrichtung 30 verfügt über drei Lagerschalenabschnitte 32, 36. Die Lagerschalenabschnitte 32 bilden äußere und nach unten gerichtete Lagerschalen 33. Der zentrische Lagerschalenabschnitt 36 bildet einen tiefer angeordneten Lagerschalenabschnitt mit nach oben gerichteter Lagerschale 37. Korrespondierend hierzu weist die Scharnierteileinrichtung 90 am Außenbauteil 52 einen zentrischen Achsträger 92 auf, dessen Unterseite eine gewölbte Lagerkontaktfläche 93 bildet, die zur Aufnahme im Lagerschalenabschnitt 36 vorgesehen ist. Beidseitig sind am Achsträger 92 frei hervorstehende Achsabschnitte 94 vorgesehen, die an ihrer Oberseite gewölbte Lagerkontaktflächen 95 aufweisen.

In Fig. 3 ist mittels des Pfeils 8 die Fügerichtung verdeutlicht, in Richtung derer die Scharnierteileinrichtung 90 in die Scharnierteileinrichtung 30 eingeschoben wird. Die Fügerichtung schließt mit der Mittelachse 2 des Basisbauteils 22 einen Winkel von etwa 90° ein.

Um die Schwenkbeweglichkeit der Betätigungseinheit 50 gegenüber der Basiseinheit 20 zu limitieren, sind beidseitig an einer Schürze 80 des Außenbauteils 52 Anschlagsflügel 82 vorgesehen. Diese Anschlagsflügel 82 sind so bemessen, dass das Außenbauteil 52 mit bereits eingefügtem Innenbauteil 54 durch die freie Öffnung an der Oberseite des Basisbauteils 22 eingesetzt werden kann, bevor es zum Zwecke der Zusammenfügung der Scharniereinrichtung 30, 90 in Richtung des Pfeils 8 verschoben wird. Im derart zusammengesetzten Zustand befinden sich die Anschlagsflügel 82 unterhalb von Halteflächen 38 des Basisbauteils 22. Wie in Fig. 4B zu erkennen ist, sind die Anschlagsflügel 82 gegenüber der Applikatorachse 4 schräggestellt, wobei sie frei zur Seite hervorragen und oberhalb und unterhalb derAnschlagsflügel 82 keine weiteren Abschnitte des Außenbauteils 52 vorhanden sind.

Neben der Funktion der Anschlagsflügel 82, im Betrieb das Rückschwenken der Betätigungseinheit 50 gegenüber der Basiseinheit 20 zu begrenzen, erfüllen sie eine Funktion bei der Montage, die im Weiteren noch erläutert wird.

Als Originalitätsschutz weist der Austragkopf 10 einen Betätigungsschutz 40 auf. Dieser ist in Form einer von der ringförmigen Außenwandung des Basisbauteils 22 nach innen weisenden Schutzabschnitt 42 vorgesehen, der eine T-Form aufweist. Von diesem Schutzabschnitt 42 erstrecken sich versetzt zur zentrischen Anbindung des Schutzabschnittes an der ringförmigen Außenwandung und oberhalb dieser Anbindung zwei zerstörbare Brückenabschnitte 44 ebenfalls zur ringförmigen Außenwandung. Diese Brückenabschnitte 44 sind derart platziert, dass die Schürze 80 beim ersten Niederdrücken der Betätigungseinheit 50 diese Brückenabschnitte 44 durchtrennt, wobei sich aufgrund der hiervon nicht betroffenen zentralen Anbindung des Schutzabschnittes hierbei kein Teil von dem Basisbauteil 22 löst.

Der Betätigungsschutz 40 dient vorliegend primär der Verhinderung einer unbeabsichtigten Betätigung des Flüssigkeitsspenders, beispielsweise im Reisegepäck. Er kann zusätzlich auch dem Zweck dienen, visuell erkennen zu können, ob der Flüssigkeitsspender bereits in Betrieb genommen wurde und ein erster Austrag erfolgt ist. Dies kann beispielsweise erreicht werden, indem ein Spalt zwischen der Schürze 80 und der Ringwandung des Basisbauteils 22 ausreichend groß ist, dass die Brückenabschnitte 44 vor Erstverwendung durch diesen Spalt sichtbar sind.

Fig. 5 verdeutlicht eine besondere Funktion der Scharniereinrichtung 30, 90. Die Formgebung der Lagerschalen 33, 37 ist so gewählt, dass der Achsträger 92 und die Achsabschnitte 94 und damit die Betätigungseinheit 50 in Richtung des Pfeils 9 und somit entsprechend der Fügerichtung 8 eine Beweglichkeit bewahren, vorliegend um 0,3 mm, ohne dass hierfür eine Verformung eines der Bauteile 22, 52 stattfindet. Diese Beweglichkeit wirkt der Gefahr entgegen, dass der Anschlussstutzen 56 bei der Betätigung des Auslassventils 112 verkantet.

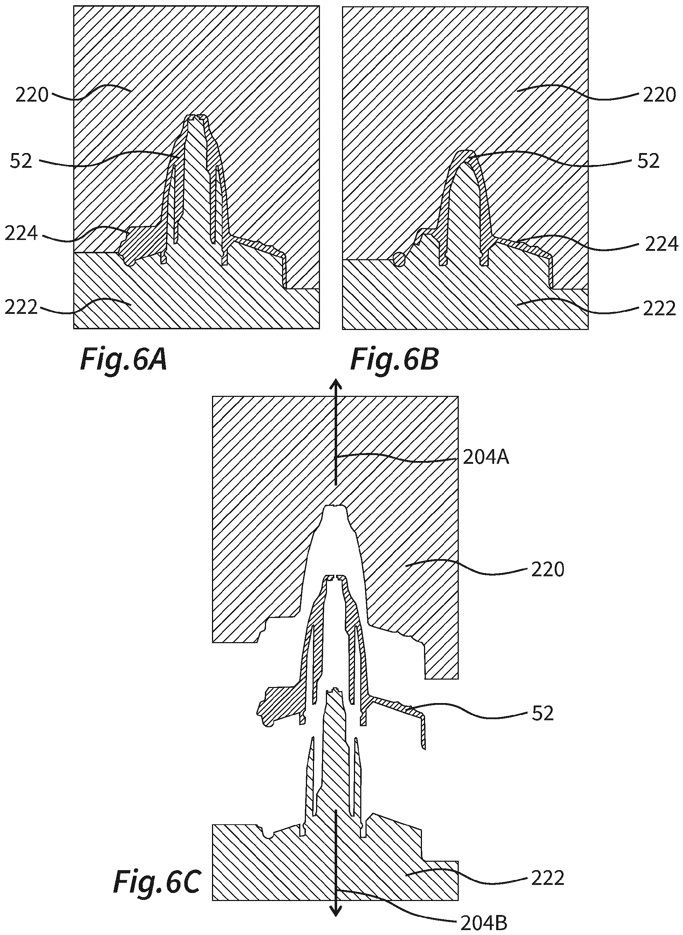

Fig. 6A bis 6C verdeutlichen den Spritzgussvorgang zur Herstellung des Außenbauteils 52. Der Spritzgussvorgang erfolgt mittels einer einachsigen Spritzgussform, bestehend aus zwei Spritzgussformsegmenten 220, 222, die gemeinsam eine Kavität 224 definiert.

Die Fig. 6A und 6B zeigen die Spritzgussform während des Spritzgießens in zwei unterschiedlichen Ebenen. Fig. 6A zeigt eine mittige Schnittebene durch den Achsträger 92 hindurch. Fig. 6B zeigt eine versetzte Schnittebene durch einen der Achsabschnitte 94 hindurch. Es ist zu erkennen, dass die beiden Spritzgussformsegmente 220, 222 jeweils die Oberseite und die Unterseite dieser Teilabschnitte definieren.

Fig. 6C zeigt den Vorgang des Trennens der Spritzgussformsegmente 220, 222 und damit der Entformung des Außenbauteils 52. Es ist zu erkennen, dass die Spritzgussformsegmente 220, 222 in einer einheitlichen Trennrichtung 204A, 204B getrennt werden. Weitere Relativbewegungen von Schiebern oder dergleichen sind nicht erforderlich.

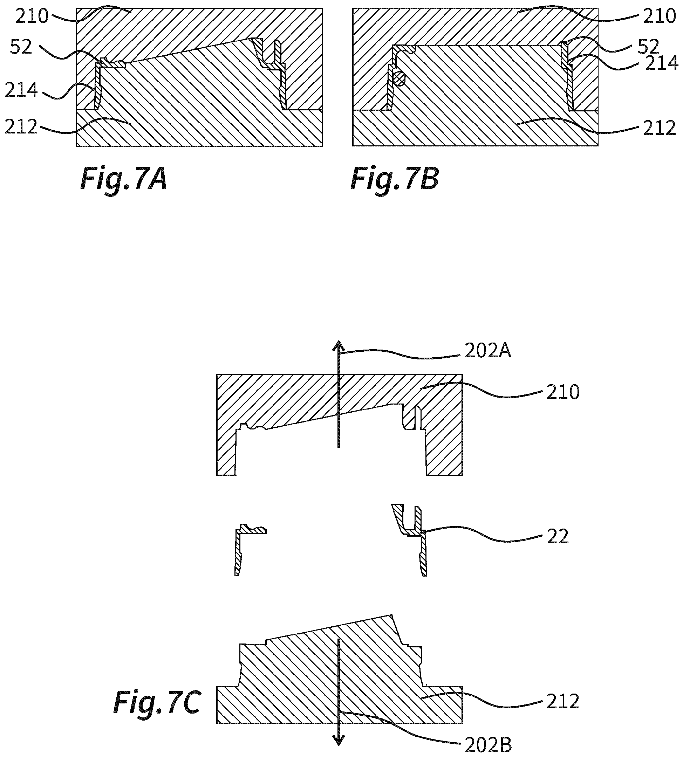

Analog zu den Figuren 6A bis 6C zeigen die Figuren 7A bis 7C die Herstellung des Basisbauteils 22. Der Spritzgussvorgang erfolgt mittels einer einachsigen Spritzgussform, bestehend aus zwei Spritzgussformsegmenten 210, 212, die gemeinsam eine Kavität 214 definiert.

Die Fig. 7A und 7B zeigen die Spritzgussform während des Spritzgießens in zwei unterschiedlichen Ebenen. Fig. 7A zeigteine mittige Schnittebene durch den zentrischen Lagerschalenabschnitt36 hindurch. Fig. 7B zeigt eine versetzte Schnittebene durch einen der Lagerschalenabschnitte 32 hindurch. Es ist zu erkennen, dass die beiden Spritzgussformsegmente 210, 212 jeweils die Oberseite und die Unterseite dieser Lagerschalenabschnitte definieren.

Fig. 7C zeigt den Vorgang des Trennens der Spritzgussformsegmente 210, 212 und damit der Entformung des Basisbauteils 22. Es ist zu erkennen, dass auch hier die Spritzgussformsegmente 210, 212 in einer einheitlichen Trennrichtung 202A, 202B getrennt werden. Weitere Relativbewegungen von Schiebern oder dergleichen sind auch hier nicht erforderlich.

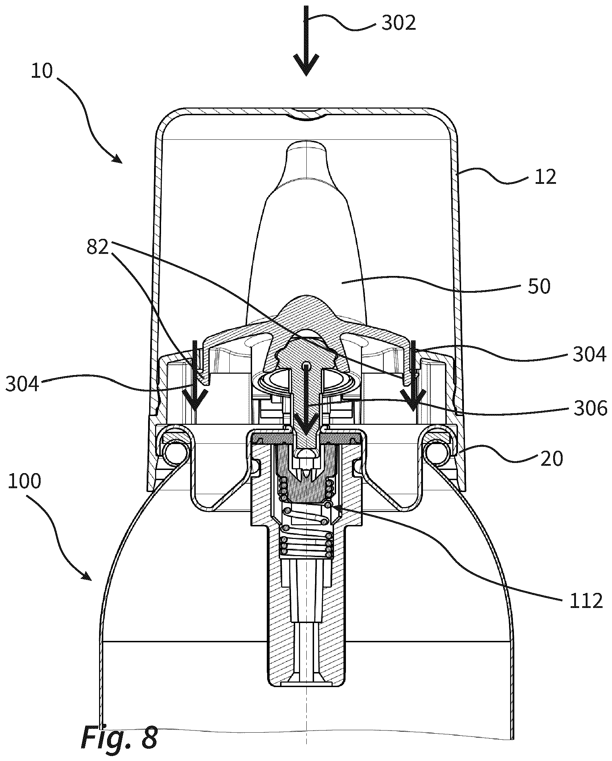

Fig. 8 verdeutlicht ein Verfahren zur Montage des Austragkopfes 10 auf einem Druckspeicher 110. Der Austragkopf 10 mitsamt einer Schutzkappe 12 wird von oben auf den Druckspeicher 110 aufgedrückt, wobei die Montagekraft in Richtung des Pfeils 302 in die Schutzkappe 12 eingebracht wird. Diese Montagekraft drückt die Kopplungseinrichtung 24 der Basiseinheit 20 über die Rastkante 122. Gleichzeitig wirkt sie über die Anschlagsflügel 82 sowie die Scharniereinrichtung 30, 90 auf die Betätigungseinheit 50, wie durch die Pfeile 304 verdeutlicht ist. Damit wirkt die Montagekraft mittelbar über die Anschlagsflügel 82 auf den Anschlussstutzen 56, der in das Auslassventil 112 hineingedrückt wird, wie durch Pfeil 306 verdeutlicht wird.

## Patentansprüche

1. Austragkopf (10) für die nasale Applikation von Flüssigkeit aus einem Druckspeicher (110) mit den folgenden Merkmalen:
a. der Austragkopf (10) verfügt über eine Basiseinheit (20) mit einer Kopplungseinrichtung (24), mittels derer der Austragkopf (10) am Druckspeicher (110) befestigbar ist, und
b. der Austragkopf (10) verfügt über eine Betätigungseinheit (50), die einen in einer Applikatorachse (4) erstreckten länglichen Nasalapplikator (60) und eine Betätigungsfläche (70) aufweist,
c. die Betätigungseinheit (50) ist mittels einer Scharniereinrichtung (30, 90) um eine Schwenkachse (6) schwenkbar an der Basiseinheit (20) angebracht, so dass die Betätigungseinheit (50) zum Zwecke des Öffnens eines Auslassventils (112) des Druckspeichers (110) in einer schwenkenden Betätigungsrichtung niederdrückbar ist.

2. Austragkopf (10) nach Anspruch 1 mit dem folgenden weiteren Merkmal:
a. die Schwenkachse (6) und die Betätigungsfläche (70) sind gegenüber dem Nasalapplikator (60) an entgegengesetzten Seiten der Betätigungseinheit (50) angeordnet.

3. Austragkopf (10) nach Anspruch 1 oder 2 mit den folgenden weiteren Merkmalen:
a. die Basiseinheit (20) weist ein Basisbauteil (22) auf, welches mindestens die Kopplungseinrichtung (24) sowie eine basiseinheitsseitige Scharnierteileinrichtung (30) umfasst, und
b. das Basisbauteil (22) weist eine Bauteilgeometrie auf, die bezogen auf eine Trennrichtung, die einer Mittelachse (2) der Basiseinheit (20) entspricht, frei von nicht zwangsentformbaren Hinterschneidungen ist.

4. Austragkopf (10) nach einem der vorstehenden Ansprüche mit dem folgenden weiteren Merkmal:
a. die Basiseinheit (20) weist eine basiseinheitsseitige Scharnierteileinrichtung (30) auf, die mindestens zwei äußere Lagerschalenabschnitte (32) und unterhalb der äußeren Lagerschalenabschnitte (32) und bezogen auf eine Querrichtung zur Applikatorachse zwischen äußeren Lagerschalenabschnitten (32) einen inneren Lagerschalenabschnitt (36) aufweist, vorzugsweise mit dem folgenden zusätzlichen Merkmal:
b. die Basiseinheit (20) weist ein Basisbauteil (22) auf, an dem die basiseinheitsseitige Scharnierteileinrichtung (30) vorgesehen ist, wobei zum Zwecke der Entformbarkeit des Basisbauteils (22) bei der Spritzgussherstellung bezogen auf die Mittelachse (2) oberhalb des inneren Lagerschalenabschnitts (32) und unterhalb der äußeren Lagerschalenabschnitte (36) keine Abschnitte des Basisbauteils (22) vorhanden sind.

5. Austragkopf (10) nach einem der vorstehenden Ansprüche mit den folgenden weiteren Merkmalen:
a. die Betätigungseinheit (50) weist ein Außenbauteil (52) auf, welches mindestens eine betätigungseinheitsseitige Scharnierteileinrichtung (90) und die Betätigungsfläche (70) umfasst, und
b. das Außenbauteil (52) weist eine Bauteilgeometrie auf, die bezogen auf eine Trennrichtung, die der der Applikatorachse (4) entspricht, frei von nicht zwangsentformbaren Hinterschneidungen ist.

6. Austragkopf (10) nach einem der vorstehenden Ansprüche mit den folgenden weiteren Merkmalen:
a. die Betätigungseinheit (50) weist eine betätigungseinheitsseitige Scharnierteileinrichtung (90) auf, und
b. die Scharnierteileinrichtung (90) weist einen zentralen Achsträger (92) auf, der an seiner Unterseite über eine Lagerkontaktfläche (93) verfügt, und
c. die Scharnierteileinrichtung (90) weist beidseitig am Achsträger (92) einander gegenüberliegend zwei Achsabschnitte (94) auf, die an ihrer Oberseite über eine Lagerkontaktfläche (95) verfügen,
vorzugsweise mit dem folgenden zusätzlichen Merkmal:
d. die Betätigungseinheit (50) weist ein Außenbauteil (52) auf, an dem die betätigungseinheitsseitige Scharnierteileinrichtung (90) vorgesehen ist, wobei zum Zwecke der Entformbarkeit des Außenbauteils (52) bei der Spritzgussherstellung bezogen auf die Applikatorachse (4) oberhalb der Oberseite der Achsabschnitte (94) und unterhalb der Unterseite des Achsträgers (92) keine Abschnitte des Außenbauteils (52) vorhanden sind.

7. Austragkopf (10) nach einem der vorstehenden Ansprüche mit dem folgenden weiteren Merkmal:
a. die Betätigungseinheit (50) weist an einer der Scharniereinrichtung (90) abgewandten Seite eine Schürze (80) auf, die sich in Richtung der Applikatorachse (4) erstreckt.

8. Austragkopf (10) nach einem der vorstehenden Ansprüche mit dem folgenden weiteren Merkmal:
a. die Betätigungseinheit (50) weist mindestens einen nach außen ragenden Anschlagsflügel (82) auf, der die Beweglichkeit der Betätigungseinheit (50) entgegen der Betätigungsrichtung durch Anschlagen an einer korrespondierenden Haltefläche der Basiseinheit (20) limitiert,
vorzugsweise mit mindestens einem der folgenden zusätzlichen Merkmale:
b. die Betätigungseinheit (50) weist mindestens zwei Anschlagsflügel (82) auf, die einander gegenüberliegend an beiden Seiten der Betätigungseinheit (50) vorgesehen sind, und/oder
c. eine Anschlagsfläche des mindestens einen Anschlagsflügel (82) ist derart ausgerichtet, dass ein Normalenvektor auf der Anschlagsfläche gegenüber der Applikatorachse (4) schräggestellt ist, und/oder
d. die Betätigungseinheit (50) weist ein Außenbauteil (52) auf, an dem der mindestens eine Anschlagsflügel (82) vorgesehen ist, wobei zum Zwecke der Entformbarkeit desAußenbauteils bezogen auf die Applikatorachse (4) oberhalb und unterhalb der des Anschlagsflügels keine Abschnitte des Außenbauteils (52) vorhanden sind.

9. Austragkopf (10) nach einem der vorstehenden Ansprüche mit den folgenden weiteren Merkmalen:
a. die Scharniereinrichtung (30, 90) weist eine erste Scharnierteileinrichtung (30) und eine zweite Scharnierteileinrichtung (90) an der Basiseinheit (20) und der Betätigungseinheit (50) auf, und
b. die erste Scharnierteileinrichtung (30) bildet ein Scharnierlager und weist mindestens zwei äußere Lagerschalenabschnitte (32) und einen hierzu in Richtung der Applikatorachse (4) gegenüberliegend angeordneten inneren Lagerschalenabschnitt (36) auf, und
c. die zweite Scharnierteileinrichtung (90) bildet eine Scharnierachse und weist einen zentrischen Achsträger (92) auf, der am inneren Lagerschalenabschnitt (36) gelagert ist sowie zwei hiermit fluchtende frei hervorstehende Achsabschnitte (94), die an den äußeren Lagerschalenabschnitten (32) gelagert sind.

10. Austragkopf (10) nach einem der vorstehenden Ansprüche mit den folgenden weiteren Merkmalen:
a. die Scharniereinrichtung (30, 90) weist eine erste Scharnierteileinrichtung (30) an der Basiseinheit (20) und eine zweite Scharnierteileinrichtung (90) an der Betätigungseinheit (50) auf, und
b. eine Fügerichtung (8) der Scharniereinrichtung (30, 90), in Richtung derer die Scharnierteileinrichtungen (30, 90) der Betätigungseinheit (50) und der Basiseinheit (20) koppelbar sind, ist gegenüber einer Mittelachse (2) der Basiseinheit (20) angewinkelt, vorzugsweise um einen Winkel von mindestens 60°, insbesondere vorzugsweise um einen Winkel von mindestens 80°,
vorzugsweise mit dem folgenden weiteren Merkmal:
c. die Scharnierteileinrichtungen (30, 90) sind derart geformt, dass sie in einem gekoppelten Zustand verformungsfrei gegeneinander in Fügerichtung (8) begrenzt beweglich sind, vorzugsweise um eine Verlagerungsstrecke von mindestens 0,2 mm.

11. Austragkopf (10) einem der vorstehenden Ansprüche mit den folgenden weiteren Merkmalen:
a. der Austragkopf (10) weist einen Betätigungsschutz (40) auf, der einen an der Betätigungseinheit oder der Basiseinheit (20) einstückig angeformten Schutzabschnitt (42) umfasst, und
b. der Schutzabschnitt (42) weist mindestens einen zerstörbaren Teilabschnitt (44) auf, der durch eine Betätigungsverlagerung der Betätigungseinheit (50) gegenüber der Basiseinheit (20) zerstörbar ist,
vorzugsweise mit mindestens einem der folgenden zusätzlichen Merkmale:
c. der Schutzabschnitt (42) weist mindestens zwei zerstörbare Teilabschnitte (44) auf, und/oder
d. der Schutzabschnitt (42) ist derart ausgebildet, dass nach Zerstörung des mindestens einen zerstörbaren Teilabschnitts (44) keine Trennung eines Teiles des Schutzabschnitts (42) von der Betätigungseinheit bzw. der Basiseinheit (20) eintritt, wobei der Schutzabschnitt (42) nach Zerstörung des mindestens einen Teilabschnitts vorzugsweise fest an der Basiseinheit (20) verbleibt.

12. Austragkopf (10) nach einem der vorstehenden Ansprüche mit den folgenden zusätzlichen Merkmalen:
a. die Betätigungseinheit (50) weist ein Außenbauteil (52) auf, welches mindestens die Betätigungsfläche (70), die betätigungseinheitsseitige Scharnierteileinrichtung (90) sowie einen Hüllenabschnittdes Nasalapplikators (60) einschließlich einer Austragöffnung (62) bildet, und
b. die Betätigungseinheit (50) weist ein Innenbauteil (54) auf, welches mit dem Außenbauteil (52) dauerhaft verbunden ist und einen Leitungsabschnitt bildet, mittels dessen Flüssigkeit vom Druckspeicher zur Austragöffnung geleitet werden kann.

13. Austragkopf (10) nach einem der vorstehenden Ansprüche mit mindestens einem derfolgenden zusätzlichen Merkmalen:
a. die eine Applikatorachse (4) ist gegenüber einer Mittelachse (2) der Basiseinheit (20) schräggestellt, wobei bezogen auf einen unbetätigten Zustand der Winkel zwischen der Mittelachse (2) und der Applikatorachse (4) vorzugsweise mindestens 10° beträgt, insbesondere vorzugsweise mindestens 15°, und/oder
b. mindestens ein im Betrieb sichtbares Bauteil der Betätigungseinheit und mindestens ein im Betrieb sichtbares Bauteil der Basiseinheit weisen eine unterschiedliche Farbgebung auf, und/oder
c. der Austragkopf verfügt über eine Schutzkappe, die auf der Basiseinheit aufrastbar ist, wobei vorzugsweise eine Führungsfläche an der Basiseinheit, im Bereich derer die Schutzkappe gestützt wird, in Richtung der Mittelachse eine Erstreckung von mindestens 5 mm aufweist, vorzugsweise von mindestens 8 mm.

14. Flüssigkeitsspender (100) zum Austrag von Flüssigkeiten mit dem folgenden Merkmal:
a. der Flüssigkeitsspender (100) weist einen Druckspeicher (110) sowie einen hieran befestigten Austragkopf (10) auf,
**gekennzeichnet durch** das folgende zusätzliche Merkmal:
b. der Austragkopf (10) ist nach einem der vorstehenden Ansprüche ausgebildet.

15. Verfahren zur Herstellung eines Basisbauteils (22) einer Basiseinheit (20) für einen Austragkopf (10) nach einem der Ansprüche 1 bis 13 mit den folgenden Merkmalen:
a. das Basisbauteil (22) weist eine basiseinheitsseitige Scharnierteileinrichtung (30) auf, wobei die Scharnierteileinrichtung (30) über mindestens einen unteren und mindestens einen oberen Lagerschalenabschnitt (32, 36) verfügt, und
b. die Herstellung des Basisbauteils (22) erfolgt mittels eines Spritzgussverfahrens, bei dem eine Mehrzahl von Spritzgussformsegmenten (210, 212) Verwendung findet, die entlang einer gemeinsamen Öffnungs- und Trennrichtung (202A, 202B) verlagerbar sind, und
c. im Zuge der Herstellung werden die Spritzgussformsegmente (210, 212) zur Bildung einer dem Basisbauteil entsprechenden Kavität zusammengefahren, Kunststoffmaterial wird in die Kavität eingespritzt und nach dem Aushärten werden die Spritzgussformsegmente zu m Zwecke der Entformung in der gemeinsamen Öffnungs- und Trennrichtung (202A, 202B) gegeneinander verlagert,
vorzugsweise mit dem folgenden weiteren Merkmal:
d. zum Zwecke der Entformung wird mindestens ein erstes Spritzgussformsegment (210), welches an eine Oberseite des mindestens einen oberen Lagerschalenabschnitts (32) und an eine obere Innenseite des mindestens einen unteren Lagerschalenabschnitts (36) des Basisbauteils (22) angrenzt, gegenüber mindestens einem zweiten Spritzgussformsegment (220), welches an eine Unterseite des mindestens einen unteren Lagerschalenabschnitts (36) und eine untere Innenseite des mindestens einen oberen Lagerschalenabschnitts (32) angrenzt, beabstandet.

16. Verfahren zur Herstellung eines Außenbauteils (52) einer Betätigungseinheit (50) für einen Austragkopf (10) nach einem der Ansprüche 1 bis 13 mit den folgenden Merkmalen:
a. das Außenbauteil (52) weist eine betätigungseinheitsseitige Scharnierteileinrichtung (90) auf, wobei die Scharnierteileinrichtung (90) über einen zentralen Achsträger (92) verfügt, der an seiner Unterseite eine Lagerkontaktfläche (93) aufweist, und wobei die Scharnierteileinrichtung (90) beidseitig am Achsträger (92) einander gegenüberliegend zwei Achsabschnitte (94) aufweist, die an ihrer Oberseite über eine Lagerkontaktfläche (95) verfügen,
b. die Herstellung des Außenbauteils (52) erfolgt mittels eines Spritzgussverfahrens, bei dem eine Mehrzahl von Spritzgussformsegmenten (220, 222) Verwendung findet, die entlang einer gemeinsamen Öffnungs- und Trennrichtung (204A, 20B) verlagerbar sind, und
c. im Zuge der Herstellung werden die Spritzgussformsegmente (220, 222) zur Bildung einer dem Außenbauteil (52) entsprechenden Kavität zusammengefahren, Kunststoffmaterial wird in die Kavität eingespritzt und nach dem Aushärten werden die Spritzgussformsegmente zum Zwecke der Entformung in der gemeinsamen Öffnungs- und Trennrichtung (204A, 204B) gegeneinander verlagert,
vorzugsweise mit dem folgenden zusätzlichen Merkmal:
d. zum Zwecke der Entformung wird mindestens ein erstes Spritzgussformsegment (220), welches an eine Oberseite der der Achsabschnitte (94) angrenzt, gegenüber mindestens einem zweiten Spritzgussformsegment (222), welches an eine Unterseite des zentralen Achsträgers (92) angrenzt, beabstandet.

17. Verfahren zur Anbringung eines Austragkopfes (10), insbesondere nach einem der Ansprüche 1 bis 13, mit einer Basiseinheit (20) und einer Betätigungseinheit (50) auf einem Druckspeicher (110) mit den folgenden Merkmalen:
a. die Betätigungseinheit (50) verfügt über mindestens einen nach außen ragenden Anschlagsflügel (82) sowie einen Anschlussstutzen zur Kraftübertragung auf ein Auslassventil des Druckspeichers (110), wobei der mindestens eine Anschlagsflügel (82) die Beweglichkeit der Betätigungseinheit (50) entgegen der Betätigungsrichtung durch Anschlagen an einer korrespondierenden Haltefläche der Basiseinheit (20) limitiert, und
b. zum Zwecke des Aufschnappens des Austragkopfes (10) an einer Rastkante (122) des Druckspeichers (110) wird der Austragkopf (10) an der Basiseinheit (20) in Richtung des Druckspeichers (110) mit einer Montagekraft kraftbeaufschlagt, und
c. die Fügekraft wird zum Teil über den mindestens einen Anschlagsflügel (82) auf die Betätigungseinheit (50) übertragen, um das Koppeln des Anschlussstutzens an das Auslassventil des Druckspeichers (110) zu gewährleisten.
